(19) **Europäisches Patentamt / European Patent Office / Office européen des brevets**

(11) **EP 1 458 495 B1**

(12) **EUROPEAN PATENT SPECIFICATION**

(45) Date of publication and mention
of the grant of the patent:
**02.07.2008 Bulletin 2008/27**

(21) Application number: **02793845.5**

(22) Date of filing: **29.10.2002**

(51) Int Cl.:
*B05D 1/26* (2006.01)     *B05D 7/26* (2006.01)
*A61L 101/00* (2006.01)     *A01N 59/16* (2006.01)
*B41M 3/00* (2006.01)     *C09D 5/14* (2006.01)
*D21H 21/36* (2006.01)     *B05D 1/28* (2006.01)
*B65D 81/28* (2006.01)     *A01N 25/34* (2006.01)

(86) International application number:
**PCT/US2002/034650**

(87) International publication number:
**WO 2003/039766 (15.05.2003 Gazette 2003/20)**

(54) **ANTI-MICROBIAL PACKAGING MATERIALS AND METHODS FOR MAKING THE SAME**

ANTIMIKROBIELLE VERPACKUNGSMATERIALIEN UND VERFAHREN ZUR HERSTELLUNG DERSELBEN

MATERIAUX D'EMBALLAGE ANTIMICROBIENS ET LEURS PROCEDES DE FABRICATION

(84) Designated Contracting States:
**AT BE BG CH CY CZ DE DK EE ES FI FR GB GR IE IT LI LU MC NL PT SE SK TR**

(30) Priority: **02.11.2001 US 3799**

(43) Date of publication of application:
**22.09.2004 Bulletin 2004/39**

(73) Proprietor: **BPSI Holdings, Inc.**
**Wilmington, Delaware 19899-8985 (US)**

(72) Inventor: **PODHAJNY, Richard, M.**
**Collegeville, PA 19046 (US)**

(74) Representative: **Pizzoli, Pasquale Vincenzo et al**
**c/o Società Italiana Brevetti**
**Via G. Carducci 8**
**20123 Milano (IT)**

(56) References cited:
**FR-A- 2 811 304**     **US-A- 4 859 733**
**US-A- 4 938 958**     **US-A- 5 556 699**
**US-B1- 6 231 953**

**Description**

## FIELD OF THE INVENTION

The present invention pertains to a novel, inexpensive method for placing an anti-microbial coating onto packaging materials and to polymer dispersions containing anti-microbial zeolites. The stable, zeolite-containing dispersions may be formulated in water-based or solvent-based systems. They are of particular importance for use on food packaging films.

## BACKGROUND OF THE INVENTION

Anti-microbial packaging is of increasing importance in the food industry. Anti-microbial packaging allows manufacturers to distribute products with longer shelf lives, which permits the manufacturer to decrease distribution costs. Anti-microbial packaging also enables both the merchant and the consumer to stockpile products. This permits the manufacturer to increase sales volume. Anti-microbial packaging also provides some assurance against intentional and unintentional contamination. Additionally, it is speculated that manufacturers who utilize anti-microbial packaging will see their products purchased in preference to other brands which lack anti-microbial packaging. Lastly, the ability to protect foods longer may permit a transition to fresher food products, transforming the market and establishing new brands.

Various anti-microbial agents have been used with packaging materials. Examples of anti-microbial agents used in paper and plastic food packaging materials include alcohols and organic acids such as acetic, proprionic, benzoic and sorbic acids. Organic acid salts have been employed as well. Examples include potassium and calcium sorbates, sodium benzoate and quaternary salts. Organic acid anhydrides such as sorbic acid anhydride, and benzoic acid anhydrides have also been employed. Polymeric anti-microbial materials such as hexyl-PVP have also been suggested for use as packaging materials. Another suggested category of anti-microbials are inorganic anti-microbials, examples of which include sulfites, nitrites, chlorides, carbon dioxide, sulphur dioxide and silver salts.

These anti-microbial materials have been added to packaging materials in a variety of ways. In some of the simplest applications, anti-microbials have been dusted in or sprayed on packaging materials. However, anti-microbial agents that are not attached to the packaging material may leech from the package surface. For food products in particular, leeching of the anti-microbial agent is not desirable as it may lead to ingestion of the anti-microbial. These anti-microbial treatments are also subject to wear and typically lose effectiveness with time and handling. For long-term anti-infective properties, it has been suggested that the anti-microbial agent be incorporated into the raw material from which the packaging material will be made. In so doing, however, much of the anti-microbial agent is "buried" within the packaging material. Though the buried anti-microbial will "ride with" the package, and thus serve some preventative role, it will not be available for the first line of defense at the packaging surface. The amount of buried, wasted anti-microbial agent in molded or extruded packaging materials is especially high.

One of the difficulties encountered with producing anti-microbial or microbially resistant packaging materials is the cost of the anti-microbial agent. Anti-microbial agents, typically, are more expensive than the paper and/or plastic material that forms the remainder of the packaging. Another difficulty is the cost associated with incorporating the specific anti-microbial agent into the packaging material. For certain products, particularly food products, the cost of anti-microbial packaging materials has been prohibitive. With the growing emphasis on the importance of anti-microbial properties, longer-acting, more expensive anti-microbial agents are being developed. Zeolites are a preferred, long-acting anti-microbial agent, but they are quite expensive. It would be highly desirable to be able to use zeolites as part of a more cost effective anti-microbial coating.

It has been suggested to use anti-microbial zeolites to render polymeric resins anti-microbial. Specifically, U.S. Patent No. 4,938,958 discloses at column 4, beginning at line 34, "incorporating the *antibiotic* zeolite into the resin by means of kneading it with the zeolite or coating the *antibiotic* zeolites on the surface of such a resin" (emphasis added). No further description of coating is offered. No level of addition is offered. It is not stated that the addition is made to molten resin, or with wet or dry zeolites. No suggestion of forming a coating solution is given. This patent also suggests directly mixing zeolites with paints to impart *antibiotic* properties, "or by coating the zeolite on the surface of the coated films" (emphasis added) (col. 4., lines 56-65). Again, there is no description of how to coat and no instruction as to how to convert the paints to formulations that can be easily and inexpensively combined with packaging materials, such as clear plastic film, to render them anti-microbial.

Recently, there have been suggestions to put silver-containing zeolites into plastic films, which are a preferred packaging material for food. For example, in Food Contact Substance Notification FCN 000047, the Food and Drug Administration has approved Zeolite A made by Sinanen Company Ltd. for use in all types of food contacting polymers, in a level of up to 5%. However, films are typically made by extrusion processes, and for the reasons given above, anti-microbial agents may be buried in an extrusion product. This is especially true for films, as the heat which causes the flow of the film-former also creates a skin of film-former at the surface, which blocks the anti-microbial agent from the surface. However, packaging films are water-repellant (hydrophobic) and are typically produced by the extrusion process, and for the reasons given above, anti-microbial agents may be buried within the hydrophobic product. Since the performance of the anti-microbial agent depends on mobility through a moisture medium, the extruded hydrophobic polymer limits the anti-microbial effectiveness since it reduces the anti-microbial mobility. Thus, a substantial amount of the zeolite near the surface is covered and therefore unavailable for its intended purpose. The result is a film that is expensive to manufacture and less effective than desired.

One specific attempt to incorporate anti-microbial zeolites into films and the like is found in U.S. Patent No. 5,556,699, (the '699 patent). The '699 patent discloses preparing "antibiotic" films by admixing the zeolites and a variety of polymer materials (see col. 4, lines 24-44) in the usual manner and forming the films by any known method such as casting, extrusion (inflation, T-die, calendering, cutting), and drawing methods (see col. 4 lines 45-58). In addition, the patent discloses laminates made from such films, by co-extrusion, or laminating (col. 5, lines 12-14). Examples 1-3 and 5 demonstrate co-extrusion. Example 4 describes using a mixture of polyurethane and zeolites to coat a substrate used in the manufacture of a toothbrush, prior to the addition of the bristles. See col. 9, line 26. Despite of this disclosure, further improvements in coating methods and in zeolite coating formulations have been sought. For example, in the past, it was known that the zeolites would rather quickly settle at the bottom of the vessel. Thus, the actual coating applied to the surface would often contain far less of the anti-microbial than desired. The effectiveness of the anti-microbial activity was also less than expected.

More sophisticated mechanisms for incorporation of anti-microbials into packaging are also being developed. US. Patent No. 6,264,936 B1 discloses a non-leeching, long acting, anti-microbial coating, which kills microorganisms on contact. The coating has particular importance for the inside surface of bottles containing ocular solutions. The coating comprises a polymer matrix made up of arms or tentacles of the polymer, and a biocide contained in reservoirs held within a swirl of tentacles, and attached, one molecule at a time, to the tentacles. The polymer material "must be capable of insinuating the biocide into the cell membrane of the microorganism". Thus, the biocide is released "into the microorganism but not into the surrounding environment" (See col. 2, lines 49-59).

Packaging materials are typically made by converters, who construct the materials from existing paper or plastic stock. Converters typically run printing, scoring, laminating and folding operations, which operate at room temperature. Their profits depend on their application of these processes to the starting materials. Imparting anti-microbial properties to packaging materials has heretofore required additional production machinery, such as heated extrusion equipment. In spite of the desire of their customers to have packaging materials with long-term anti-microbial properties, converters have been unable to deliver such a product at an economical price especially for those using zeolites. Even though anti-microbial packaging commands a higher price, it has been cost prohibitive to date for converters to add the necessary equipment to produce the desired anti-microbial packaging materials. With the present invention, this advantage may be economically realized on existing converter equipment, while maintaining other expected properties in packaging materials, such as scratch resistance and handling resistance.


## SUMMARY OF THE INVENTION

In accordance with one preferred aspect of the invention there is provided a method of applying an anti-microbial treatment to the surface of a packaging material. The method includes providing a substantially inert dispersion comprising a polymer and anti-microbial zeolites, preferably a zeolite containing silver ions, printing the dispersion onto the packaging material surface and drying the dispersion to form a coating layer having the polymer and zeolites on at least a portion of the exposed surface thereof.

The zeolites comprise from about 0.5 % to about 10% by weight of the dispersion and preferably have a particle size of between about 2 and about 5 microns, a pore size of between about 3 and about 5 Angstroms. Packaging materials prepared by the process are also described herein.

In another aspect of the invention there is provided a packaging material having anti-microbial properties on at least one surface thereof Specifically, the packaging material has an anti-microbial coating layer printed on at least a portion of a surface thereof The coating layer includes a polymeric material and zeolites containing silver ions, which are present on at least a portion of the exposed surface of the coating layer. As with the method described above, the zeolites have a particle size of between about 2 and about 5 microns and a pore size of between about 3 and about 5 Angstroms. The zeolites comprise from about 1% to about 5% by weight of the dried coating layer.

In still further aspects of the invention, there is provided 1) a method for rendering paper or a cardboard substrate anti-microbial or otherwise more resistant to bacteria by applying a solvent-based polymer-zeolite dispersion as described herein to the substrate, and 2) a method for rendering a nylon, or polystyrene film anti-microbial or more resistant to bacteria by applying a water-based dispersion to the film.

The dispersions of the present invention may be applied using conventional printing equipment such as rotogravure printing apparatus, at ambient temperatures. As a result, the present invention provides a relatively inexpensive but quite versatile method for achieving anti-microbial coatings on packaging materials. The dispersions employed in the methods of the present invention are relatively low viscosity, enabling them to be easily handled by printing equipment. The dispersions are also very stable, yielding a uniform distribution of zeolites in the printed coating layer. In addition, the polymer/zeolite dispersions may be either water based or solvent based. While Applicants do not wish to be bound by any particular theory, it is believed that the zeolite particles contribute at least in part to the stability of the dispersion, and ensure uniform, high levels of zeolites in the coating layer.

One advantage afforded by the present invention is the fact that the artisan can more efficiently deliver an effective amount of the anti-microbial zeolite to the exterior surface of the coating layer. This is to be contrasted with the previously described zeolite containing coatings wherein the zeolites were forced below the surface and consequently unavailable for surface anti-microbial effect on the packaging materials. Indeed, although the silver forms no more than about 2.5% by weight of the preferred zeolite, the anti-microbial effective levels of silver is less than about 0.001% by weight of the dried coating.

The anti-microbial dispersion formulations of the present application may also provide scratch resistance and handling resistance, which are especially important in food contact films.

The anti-microbial dispersion formulations of the present invention may also incorporate a variety of other coating ingredients. For example, ink pigments for lithography, rotogravure printing, flexography, and offset gravure printing may be incorporated into the coating formulations. Additional anti-microbial agents such as ZnO can be included. Thus, the application methods may also include discontinuous, patterned, printing, or full cover printing, which extends continuously across a surface portion of the packaging material.

Other and further advantages of the present invention will become apparent to artisan of ordinary skill upon reading the specification and claims attached hereto with reference being made to the attached figures.

## BRIEF DESCRIPTION OF THE DRAWINGS

Figure 1 is a schematic representation of a zeolite used in the present invention.
Figure 2 is a schematic representation of a cross-section of an anti-microbial surface formed by the anchor coat of the present invention.

## DETAILED DESCRIPTION OF THE INVENTION

The solvent-based and water-based novel anti-microbial dispersion formulations are described herein in relation to food contacting films. This is in no way intended to limit the application of the dispersions and coatings achieved therewith. The use of singular terms for convenience in the description is in no way intended to be limiting. Thus, for example, a formulation described as comprising "an anti-microbial" includes reference to a formulation comprising one or more of such anti-microbials; and the description of "a packaging material with a coating" includes reference to one or a number of coatings, at least one of which may be the dried anti-microbial coating layer described herein. The invention is also not limited to the particular process steps or materials disclosed herein, as such process steps and materials may vary somewhat. The terminology employed herein is used for the purpose of describing particular embodiments only and is not intended to be limiting, since the scope of the present invention will be limited only by the appended claims and equivalents thereof

For the purposes of the present invention, "substantially inert" shall be understood to mean that the inventive dispersions and the ingredients included therein, e.g. the polymers and zeolites, etc., are not appreciably reactive with each other and do not cause or undergo significant precipitation or agglomeration. Furthermore, for this purpose, "significant" shall be understood to mean an amount greater than that which renders the coating dispersions unprintable using the apparatus described therein.

As pointed out above in the Summary of the Invention section, one preferred aspect of the invention includes a method of applying an anti-microbial treatment to a portion of a surface of a packaging material. The method includes the steps of providing a dispersion containing a polymer and zeolites containing silver ions wherein the zeolites have a particle size of between about 2 and about 5 microns, a pore size of between about 3 and about 5 Angstroms. The dispersion is then applied as a coating to at least a portion of a surface of the packaging material, preferably by printing as described in detail herein. The coating is permitted to be dried to form a coating layer having a surface in contact with the packaging material and an exposed surface which contains the polymer and zeolites on at least a portion thereof. The phrase "exposed surface" is intended to refer to the surface of the dried coating layer which becomes exposed to the environment, i.e. outer surface, and not the hidden (inner) surface that abuts the surface of the packaging material.

The liquid dispersions of the present invention preferably have a viscosity between about 10 and about 400 centipoise, or more preferably between about 200 and about 300 centipoise, at 10-25°C. However, the viscosity and rheology can be modified so that the antimicrobial ink or coating can be applied by various printing and coating methods. For example, for lithographic processes, viscosities in the range of about 50,000 centipoise would be desired.

The zeolites used in the claimed process preferably have a particle size of between about 2 and about 5 microns and a pore size of between about 3 and about 5 Angstroms. In especially preferred aspects of the invention, the zeolites have a particle size of at least about 5 microns and a pore size of at least about 4 Angstroms.

In these aspects of the invention, the zeolites make up from about 0.5% to about 10% by weight of the dispersion. Preferably, the zeolites comprise from 1% to 5% by weight of the dispersion while most preferably the zeolites comprise from about 2% to about 5% by weight of the dispersion.

## Dispersion Ingredients

### A. Polymers for Solvent-based Dispersions

A wide variety of polymers can be employed as part of the inventive dispersions. For example, a non-limiting list of the polymers that may be used in making the solvent-based formulations of the present invention include, for example, polyamides, acrylics, polyvinyl chloride, methyl methacrylates, polyurethanes, ethyl cellulose, polyvinylbutyrral, polyketones and nitro celluloses.

Generally, however, suitable polymers which are otherwise well suited for the purpose of being used as part of a film coating can be employed. Many solvent-based dispersions according to the present invention may be made with acrylic polymers. In one example, a polyamide is added to the acrylic polymer, and often a third resin is included. These formulations produce particularly adherent anti-microbial coatings. For example, they can be made to adhere to low energy polyethylene film. The solvent-based coating of the present invention also provides good adhesion to paper, nylon, and corona treated polyethylene. Films to which an anti-microbial dried coating layer of zeolites may beaded, may be alcohol based, and heat sealable, and are easily rendered non-fogging. Some particularly preferred polymers include polyamides available under the trade name UNI-REZ (from Arizona Chemical, Savannah, GA.

Additional resin binders useful in making 1% and 2% solvent-based dispersion formulations are SS Nitrocellulose methyl methacrylate copolymer and SS Nitrocellulose. Formulations of methylmethacrylate copolymerized rosin, and polyvinyl chloride resin where stable at 5% by weight zeolites, and dip coated samples of these formulations onto polystyrene and stainless steel yielded an anti-microbially effective amount of silver ions.

### B. Method of Making Solvent-based Anti-microbial Dispersions

The solvent-based dispersions may be formed as follows:

The resin is dissolved in an appropriate solvent(s). A variety of desired additives may then be added to the mixture, which is again stirred to assure uniform distribution of the ingredients. Next, zeolites are added and the mixture stirred vigorously until all ingredients are dispersed, i.e. for about another 30 minutes. The mixture is then passed through a horizontal mill which contains inert beads in the range of 0.5 - 2 mm to complete the break-up of zeolites agglomerates that form during shipping or storage, and to remove the air from the surface of the zeolite, so it may be more easily wetted and enter into the dispersion. Optionally, a wax may be added and the mixture again stirred until evenly distributed, i.e. for about 30 minutes. The product is then filtered through 10-25 micron filter and packed in suitable containers.

Such coating formulations retain great stability. Although some settling of the zeolite may occur in diverse conditions such as prolonged storage, mere stirring of the formulation before beginning the application process will easily yield a uniform dispersion of zeolites that remains stable through out the application process.

It will be understood by those of ordinary skill that the foregoing represents a general description of how the dispersions are formed. The exact amount of time required for each mixing step, for example, will depend upon several factors, including but not limited to specific resins selected, batch size, apparatus employed, optional ingredients employed, etc.

### C. Polymer for Water-based Dispersions

The printable dispersions can also be a water-based dispersion. In such instances, a non-limiting list of suitable polymers includes sulfonated polyesters, polyurethane, polyamides, maleics, shellacs and acrylics. In particularly preferred embodiments, the polymer is an acrylic emulsion such as those available under the trade name JONCRIL (from Johnson Polymer, 8310 16th Street, Sturtevant, WI). In other preferred embodiments, suitable polymers have an acid number of less than about 100 and more preferably less than about 60. In acrylic emulsions the acid number may be from about to 100 to about 300. These formulations are preferably alkaline, and preferably have a pH of greater than about 8, and more preferably greater than about 9.

D. <u>Method of Making Water-based Anti-microbial Dispersions</u>

The method of making the water-based dispersions, other than using water rather than organic solvents, is not substantially different from the steps followed to make the solvent-based dispersions of the present invention and will be apparent to the ordinary skilled artisan without undue experimentation.

The polymers for both the solvent-based and water-based formulations are chosen such that the dried coating layers are substantially hydrophobic and not easily dissolved in water. This provides for water resistance which is required in most packaging applications. The zeolites remain at the exposed surface of the coating layer and continue to provide the anti-microbial property for substantially the full life of the packaging material.

E. <u>Zeolites</u>

Zeolites are aluminosilicates. They have a crystalline structure which permits them to incorporate a variety of substances. Naturally occurring zeolites contain either sodium or calcium, or both, and are generally represented by the formula $Na_2O \cdot Al_2O_3 \cdot x\ SiO_2 \cdot x\ H_2O$. Synthetic zeolites may contain potassium, magnesium, and iron. Zeolites undergo ion exchanges and the anti-microbial zeolites used in the present invention are those in which anti-microbial metal ions have been exchanged for other ions in the zeolite. Anti-microbial zeolites release anti-microbial metal ions through the process of ion exchange and thus impart anti-microbial properties to the coating and the packaging material. The zeolites are dispersed as a fine solid in the dispersions. The most preferred antibiotic ion is $Ag^+$, however, copper, zinc, mercury, tin, lead, bismuth, cadmium, chromium and thallium ions are anti-microbial ions which may be used to create anti-microbial films according the present invention. The sodium, calcium, potassium and/or iron ions of the zeolite are exchanged for anti-microbial metal ions, e.g. silver ions, 12, ($Ag^+$), to produce an anti-microbial zeolite.

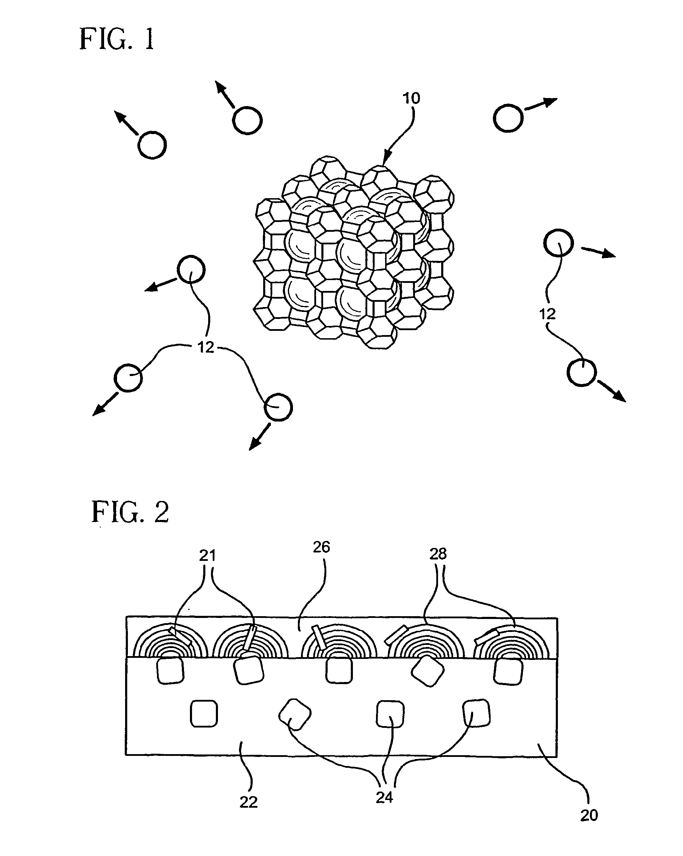

Though the remainder of the description will refer to silver-containing zeolites, it will be understood that any anti-microbial metal ion-containing zeolite may be used in the present invention. Zeolite, type A, a synthetic aluminosilicate, manufactured by Sinanen Company, Ltd, and supplied by Agion, is one particularly preferred zeolite for purposes of the present invention and is depicted as 10 in Figure 1.

In Zeolite type A, silver, zinc and ammonium ions have been exchanged for the sodium ions. The silver in the zeolite does not exceed 2.5% by weight. The free silver ions create an anti-microbial region at the surface of the coating, as shown in Figure 2, incorporating silver ions and discussed in further detail below. The zeolites used in the present application typically have a particle size of between 2 and 6 microns, and preferably between 4 and 5 microns. Most preferred are type AJ10D zeolites having a particle size of about 5 microns, and a pore size of about 4 Angstroms, permitting the silver ions to be readily released from the zeolite simply by contact with moisture. The zeolites comprise from about 1% to about 5% by weight, and preferably at least about 2% to about 5%, by weight of the dispersion.

F. **Optional Dispersion Ingredients**

The dispersions of the present invention can also contain one or more optional ingredients to improve its utility or confer additional properties to the final product. For example, preferred embodiments, the dispersion can include up to about 2 % by weight zinc oxide.

G. **Packaging Substrates for Anti-microbial Dispersion Formulation Application**

A non-limiting list of the film packaging materials that would be suitable for application of the anti-microbial dispersion formulations of the present invention include the following:

| | |
|---|---|
| Cellophanes (plain & coated) | Vinyl Chloride Co-polymers |
| Cellulose Acetate Films | Vinylidene Chloride Co-polymers |
| Ethyl Cellulose | Aluminum Foils |
| Methyl Cellulose | Laminates |
| Polyesters | Paper |
| Polyethylene | Paperboard |
| Polypropylene | Glassine |
| Polystyrene | Nylon |

Food packaging films suitable for use in the present invention include polymeric films such as blown film, oriented film, stretch and shrink film, heat shrinkable bags and food casings. "Food packaging films" as that term is used herein are flexible sheet materials which are suitably 15 mils or less and preferably less than 10 mils (25 microns) in thickness. Suitable films include regenerated cellulose and thermoplastic stretch or shrink films, and may be monolayer or multilayer films. Shrink films are preferably formed into heat shrinkable, biaxially oriented bags.

Plastics such as homopolymers or copolymers of polyolefin's e.g. polypropylene, polyethylene, or polyamides, polyethylene terphthalate, polyvinylidene chloride copolymers or ethylene-vinyl acetate copolymers may also be used to form the food-contacting films of the present invention.

There are many methods for applying the dispersion formulations of the present application, however, printing is preferred. "Printing" as defined here is the delivery of an ink or coating at the desired thickness and image pattern. Readily available printing methods include low viscosity applications, such as rotogravure, flexography, screen, pad and offset gravure, while higher viscosity applications can include offset, lithography, and roll coating. The specific printing technique to be used for the antimicrobial coating depends on the desired package material and design. Films such as polyethylene would be printed by flexography using a central impression drum to support the film web, heavy guage paper can be printed by conventional rotogravure.

Rotogravure printing is the preferred embodiment in the method for applying the dispersion coating of the present invention. Gravure processes begin with the engraving of the desired pattern or image into a plate, or about a roller. The use of a roller provides for a continuous process, printing the image repeatedly onto a moving web. Thus, in the continuous process, deemed a rotogravure process, the print image desired is carved into the surface of the roller, sometimes called a print or engraved cylinder. The printing ink or zeolite dispersion is provided in a trough. The rotating cylinder is mounted horizontally such that, a full height of the cylinder extends into the print solution in the trough. As the cylinder turns, it is flooded with print solution. A doctor blade, extending the height of the cylinder removes the excess dispersion solutions, leaving the dispersion in the carved image. The cylinder then turns to a nip with an impression roller, and prints onto a web moving through the nip. Thus, an image is placed on the web.

In the present invention, this process and equipment are used e.g. to place a pattern of the anti-microbial dispersion on a continuously moving plastic film. The trough typically contains no mechanism to stir or agitate the print solution. On occasion, such as after shipment or storage, the dispersion of the present invention may require stirring before being placed in the trough, but no subsequent stirring mechanism is required. Simply by way of illustration, and without limitation, an antimicrobial coating can be applied via rotogravure. The first requirement is to adjust the coating viscosity to allow the coating to flow out uniformly on the substrate at the desired press speed. This viscosity adjustment is typically made by the addition of solvent to the desired viscosity. To assure there is no settling of the anti-microbial additive, the anti-microbial coating is stirred for a few minutes before being pumped into the gravure coating station. The coating is then applied using an engraved rotogravure cylinder equipped with a doctor blade. Once the coating is applied at the desired thickness, it goes through a thermal drying oven which removes the solvents and produces a dried antimicrobial film.

Although the dispersions of the present invention can be used with a variety of, e.g. flexographic and rotogravure printers, some specific printers in which the dispersions can be employed without modification thereto include such press producers as Mark Andy, Comoco, Bobst-Champlain, Schiavi, PCMC, Comexi and William & Holscher.

The dispersions are particularly advantageous in printing applications, including silkscreen, offset gravure, lithographic and flexographic printing operations. More complicated or expensive equipment and processes may be used, as desired, for the dispersions are quite stable.

As stated above, films coated with the dispersion formulations of the present invention have a variety of uses, but perhaps the most important is as a food contacting film used both in food preparation and packaging, in both the commercial and home settings. However, the coating compositions of the present application have utility in any application where anti-microbial surfaces are desired. For example, the coating could be used on the surfaces of the paper inserts for food container tops or lids, or on films or paper used for disposable sanitary covers, such as those for rolling pins, or dough preparing surfaces, or plastic bags used for food storage. In some preferred aspects, the film packaging materials to which the inventive dispersion is applied include polystyrene and polyurethane.

Thus, the invention provides a novel dispersion and application method for providing an anti-microbial surface on a film, or other substrate. When dried, the dispersion yields a coating with zeolites at the surface, which will release silver ions upon the application of moisture. As shown on Figure 2, the coating composition, 20, is made up of base polymer, 22, with AgION™ powder, 24, distributed there- through. Exposure to air produces the surface film of moisture, 26, which provides for ion release at 28. The slow constant release of silver ions by the zeolite particles provides long-acting anti-microbial properties against bacteria, 21.

## EXAMPLES

The following examples serve to provide further appreciation of the invention but are not meant in any way to restrict the effective scope of the invention.

**Example 1** Aqueous Anti-microbial Coating

The following ingredients were combined to form a water-based, printable dispersion formulation according to the present invention:

| | |
|---|---|
| D.I. Water | 1.90 |
| NH4OH | 1.00 |
| Surfynol 420 | 0.10 |
| N-Propanol | 3.00 |
| Propylene glycol monomethyl ether (PGME) | 4.00 |
| Lucidene 650 | 80.0 |
| ZnO Solution | 5.00 |
| SST-3 Wax | 3.00 |
| AgION AJ 10D | 2.00 |
| | 100.00 parts by weight |

The coating formulation was prepared as follows:

1. 1.90 parts of di-ionized (D.I.) Water, 1.00 part of ammonium hydroxide solution and 0.10 parts of Surfynol 420 to 80.00 parts of Lucidene 650 were combined with stirring and stirring was continued for 30 minutes.
2. Under constant stirring, 3.00 parts of N-Propanol and 4.00 parts of PGME were added and stirring was continued for an additional 15 minutes.
3. Next, 5.00 parts of Zinc Oxide Solution and 2.00 parts of AgION's AJ 10 D were added and the ingredients were stirred vigorously for an additional 30 minutes.
4. Next, the mixture of ingredients is passed through a horizontal mill having inert beads in the range of 0.5 - 2 mm diameter.
5. Finally, 3.00 parts of SST-3 Wax were added and stirring was continued for 30 minutes.

The resultant dispersion was applied to polyethylene film via a Pamarco Hand Proofer using 180 (180 lines/ inch) anilox roll and dried in an oven at 80°C for 10 minutes. The exposed surface of the resultant anti-microbial coating layer was found to have the zeolites thereon.

**Example 2**

The treated substrates of Example 1 were next tested for anti-microbial activity against the following microbes:

| | |
|---|---|
| Salmonella | Staphylococcus |
| E. coli | Yeast |
| Pseudomonas | Mold |

These tests were performed by direct inoculation of a specific quantity of bacteria into a petri dish which contained a 2"x2" anti-microbial film sample and then quantifying the bacteria reduction using a control sample of film that did not contain the anti-microbial coating.

The quantification of the bacteria reduction in 24 hours is obtained from the following formula:

$$\% \text{ Reduction} = \text{CFU's / ml (of Assay + Control) @ T=0 - CFU's / ml}$$

$$\text{at T=24 hours CFU's/ml (of Assay+Control) @T=0.}$$

In each case, the treated surfaces were determined to be 99.9% effective against each microbe.

### Example 3

In this Example, the dispersion of Example 1 was applied to polystyrene film with #3 and #7 Meyer rods. The #3 rod produced a dry anti-microbial coating layer of about 3.75 microns, and the #7 rod, 8.75 microns. Two inch by two inch samples of the dried anti-microbial coating layer were treated with 25 ml. of 0.08% $NaNO_3$ to extract the silver ions. The #3 Meyer rods produced a sample that yielded about 503-506 mcg/L. silver ions. The #7 Meyer rods produced a sample that yielded 320 silver ions. The amount of silver ion available at the film surface is quite high and produces a very effective anti-microbial concentration. (A level of 50 $\mu$g/L of silver ions is considered a good level for anti-microbial effectiveness.)

### Examples 4 and 5

Type AJ zeolites were added to a styrenated acrylic oligomer and acrylic emulsion, at 1% (Example 4) and 2% (Example 5) by weight.

### Example 4

To an acrylic composition (FCN3359, containing Zn) was added 1% of AJ zeolite and the mixture was dispersed using a Red Devil Shaker for 6 minutes. The sample was left overnight and the dispersion had good flow properties. This sample was used to coat a test film and subsequently tested for anti-microbial effectiveness.

Reference example

### Example 5

To the acrylic composition (FGN3359, containing Zn) was added 2% of AJ zeolite and the mixture was dispersed using a Red Devil Shaker for 6 minutes. This sample was left overnight and found to have increased in viscosity to were it had little or no flow. This material could not be used for print coatings. Further tests reproduced the results.

The reason for this result is believed to be due to the fact that in this formulation the acrylic resin has an acid number above 200 is in solution, and precipitates with high levels of dissolved metal ions. Water-based formulations utilizing low acid number acrylic resins or acrylic emulsions, such as those described above, did not show the same instability with the metal ions.

While the 1% dispersion remained stable for more than 24 hours, the 2% dispersion made with the high acid number polymer produced a precipitate which seeded out, forming an almost solid mass, which could not be easily applied to plastic film or other packaging substrates at room temperatures. The increase in metal ions, with the high level of acid groups in the soluble acrylic resin, produce this precipitation. Reference example

### Example 6

On speculation that the seeding in the acrylic resin in Example 5 was due to the interaction of the silver ions with the acid groups of the acrylic resin, a 5% by weight dispersion of type AJ zeolites in sulfonated polyesters was made. Specifically, the formula for the dispersion of Example 5 was used except that the styrenated acrylic oligomer and acrylic emulsion were replaced by the sulfonated polyesters.

The resultant dispersion did not seed out. Heat was applied to accelerate any precipitation or seeding and none was noted. Thus, because of their extremely low acid number, these sulfonated polyester resins may be used to create printable formulations containing 10% by weight zeolites (dry basis).

Reference example

### Example 7 Solvent-based Anti-microbial Dispersion Formulations

The following ingredients were used to form a solvent-based dispersion according to the present invention:

| | |
|---|---|
| Polyamide Resin | 16.40 |
| N-Propyl Alcohol | 32.80 |
| D.L Water | 0.50 |
| N-Propyl Alcohol | 20.13 |

(continued)

| Nitrocellulose | 18.00 |
|---|---|
| Ethyl Acetate | 3.00 |
| Hercolyn D | 3.37 |
| Wax | 3.80 |
| AgION AJ 10D | 2.00 |
| | 100.00 parts by weight |

The dispersion was prepared as follows:

1. 16.40 parts of polyamide resin was dissolved in 32.80 parts of N-propyl alcohol that contains 0.50 parts of di-ionized (D.I.) water with stirring.

2. A solution of 18.00 parts of nitrocellulose containing 20.13 parts of N-Propanol, 3.00 Ethyl Acetate and 3.37 parts of Hercolyn D was then added to the polyamide solution and the combination was stirred well for 15 minutes.

3. Next, 2.00 parts of AgION's AJ 10 D was added to the solution under and stirred vigorously for 30 minutes.

4. The resultant mixture was passed through a horizontal mill having inert beads in the range of 0.5 - 2 mm diameter.

5. Thereafter, 3.80 parts of wax was added to the mixture and stirred for 30 minutes.

6. The coating was applied to polyethylene and tested for its ant-microbial properties. The test results showed that Direct Injection of the following bacteria showed a 99.9% reduction.

| Salmonella | Staphylococcus |
|---|---|
| E. coli | Yeast |
| Pseudomonas | Mold |

**Claims**

1. A method of applying an anti-microbial treatment to a packaging material having at least one surface, said method comprising the steps of:

   a) providing a substantially inert water-based or solvent-based dispersion comprising an acrylic polymer and zeolites containing anti-microbial metal ions, said acrylic polymer having an acid number lower than 200 when a water-based dispersion is used, said zeolites having a particle size of between 2 and 5 microns, a pore size of between 3 and 5 Angstroms, and comprising from 0.5 % to 10% by weight of the dispersion,
   b) printing said dispersion onto said surface of said packaging material, and
   c) drying said dispersion to form an antimicrobial coating layer having an exposed surface containing said polymer and said zeolites present on at least a portion thereof.

2. The method of Claim 1, wherein, the anti-microbial metal ion is a silver ion.

3. The method of Claim 1, wherein the dispersion has a viscosity between 10 and 400 centipoise at 10 -25 °C.

4. The method of Claim 1, wherein the dispersion has a viscosity between 400 and 50,000 centipoise.

5. The method of Claim 1, wherein the zeolites comprise from 1% to 5% by weight of the dispersion.

6. The method of Claim 5, wherein the zeolites preferably comprise from 2% to 5% by weight of the dispersion.

7. The method of Claim 1, wherein the dried coating layer is hydrophobic.

8. The method of Claim 1, wherein the dispersion is a solvent-based dispersion and the polymer further comprises a member selected from the group consisting of polyamides, polyvinyl chloride, methyl methacrylates, ethyl cellulose, polyvinylbutyral, polyketones and nitrocelluloses.

9. The method of Claim 1, wherein the dispersion is a water-based dispersion, and the polymer further comprises a member selected from the group consisting of sulfonated polyesters, polyamides, shellacs, and maleics.

10. The method of Claim 9, wherein the polymer further comprises a polyester.

11. The method of Claim 10, wherein the polymer further comprises a sulfonated polyester.

12. The method of Claim 8, wherein the polymer further comprises a polyamide.

13. The method of Claim 12, wherein the zeolites have a particle sizes of at least 5 microns, and a pore size of at least 4 Angstroms.

14. The method of Claim 1, wherein the dispersion is printed in a discontinuous pattern over the surface of the packaging material.

15. The method of Claim 1, wherein the printing is rotogravure printing, flexographic printing or lithographic printing.

16. The method of Claim 1, wherein the dispersion is printed on said surface at a rate of 0,163 $g/m^2$ to 3,25 $g/m^2$ (0.1 lbs./3,000 ft. square to 2 lbs./3,000 ft. square).

17. The method of Claim 1, wherein the coating layer has a thickness of from 2 microns to 20 microns.

18. The method of Claim 1, wherein the coating layer has a thickness of from 2 microns to 8 microns.

19. The method of Claim 1, wherein said packaging material is a polymer film.

20. The method of Claim 1, wherein said packaging material is selected from the group consisting of cellophanes, vinyl chlorides, vinyl chloride copolymers, cellulose acetate films, vinylidene chlorides, vinylidene chloride copolymers, ethyl cellulose, aluminum foils, methyl cellulose, laminates, polyesters, papers, polyethylenes, paperboards, polypropylenes, glassines, polystyrenes, nylons and combinations thereof.

21. The method of Claim 1 wherein the dispersion is aqueous, the polymer comprises lucidene, the zeolites have a particle size of 5 microns and a pore size of 4 Angstroms.

22. The method of Claim 1 wherein the dispersion is a solvent-based dispersion, the polymer comprises polyamides and nitrocellulose, and the zeolites have a particle size of 5 microns and a pore size of 4 Angstroms.

23. A packaging material with anti-microbial properties, obtainable by the method of Claim 1.

24. A substantially inert water-based or solvent-based dispersion of anti-microbial zeolites, said dispersion comprising an acrylic polymer and zeolites containing anti-microbial metal ions, said acrylic polymer having an acid number lower than 200 when the dispersion is water-based, said zeolites having a particle size of between 2 and 5 microns, a pore size of between 3 and 5 Angstroms, and comprising from 5% to 10% by weight of the dispersion.

25. The dispersion of Claim 24, wherein the anti-microbial metal ion is a silver ion.

26. The dispersion of Claim 24, wherein the dispersion is a solvent-based dispersion and the polymer further comprises a member selected from the group consisting of polyamides, polyvinyl chloride, methyl methacrylates, ethyl cellulose, polyvinylbutyral, polyketones and nitrocelluloses.

27. The dispersion of Claim 24, wherein the dispersion is a water-based dispersion, and the polymer further comprises a member selected from the group consisting of sulfonated polyesters, polyamides, shellacs, maleics.

**Patentansprüche**

1. Verfahren zum Anwenden einer antimikrobiellen Behandlung auf ein Verpackungsmaterial mit wenigstens einer Oberfläche, welches Verfahren die folgenden Schritte umfaßt:

   a) Zurverfügungstellen einer im wesentlichen inerten wasserbasierten oder lösungsmittelbasierten Dispersion, die ein Acrylpolymer und Zeolithe enthält, welche antimikrobielle Metallionen enthalten, wobei das Acrylpolymer eine Säurezahl kleiner als 200 hat, wenn eine wasserbasierte Dispersion verwendet wird, und wobei die Zeolithe eine Teilchengröße zwischen 2 und 5 Mikrometer haben, eine Porengröße zwischen 3 und 5 Angström haben und in einem Gewichtsanteil von 0,5 % bis 10 % in der Dispersion enthalten sind,

b) Drucken der Dispersion auf die besagte Oberfläche des Verpackungsmaterials, und

c) Trocknen der Dispersion, um eine antimikrobielle Beschichtungsschicht zu bilden, welche das Polymer und die Zeolithe enthält, die auf wenigstens einem Teil einer freien Oberfläche der Beschichtungsschicht vorhanden sind.

2. Verfahren nach Anspruch 1, bei dem das antimikrobielle Metallion ein Silberion ist.

3. Verfahren nach Anspruch 1, bei dem die Dispersion eine Viskosität zwischen 10 und 400 Zentipoise bei 10 - 25° C hat.

4. Verfahren nach Anspruch 1, bei dem die Dispersion eine Viskosität zwischen 400 und 50.000 Zentipoise hat.

5. Verfahren nach Anspruch 1, bei dem die Zeolithe in einem Gewichtsanteil von 1 % bis 5 % in der Dispersion enthalten sind.

6. Verfahren nach Anspruch 5, bei dem die Zeolithe vorzugsweise in einem Gewichtsanteil von 2 % bis 5 % in der Dispersion enthalten sind.

7. Verfahren nach Anspruch 1, bei dem die getrocknete Beschichtungsschicht hydrophob ist.

8. Verfahren nach Anspruch 1, bei dem die Dispersion eine lösungsmittelbasierte Dispersion ist und das Polymer weiter ein aus der Gruppe bestehend aus Polyamiden, Polyvinylchlorid, Methyl-Methacrylaten, Ethylzellulose, Polyvinylbutyral. Polyketonen und Nitratzellulosen ausgewähltes Element enthält.

9. Verfahren nach Anspruch 1, bei dem die Dispersion eine wasserbasierte Dispersion ist und das Polymer weiter ein aus der Gruppe bestehend aus sulfonierten Polyestern, Polyamiden, Schellacken und Maleinen ausgewähltes Element enthält.

10. Verfahren nach Anspruch 9, bei dem das Polymer weiter einen Polyester enthält.

11. Verfahren nach Anspruch 10, bei dem das Polymer weiter einen sulfonierten Polyester enthält.

12. Verfahren nach Anspruch 8, bei dem das Polymer weiter ein Polyamid enthält.

13. Verfahren nach Anspruch 12, bei dem die Zeolithe eine Teilchengröße von wenigstens 5 Mikrometer und eine Porengröße von wenigstens 4 Angström haben.

14. Verfahren nach Anspruch 1, bei dem die Dispersion in einem diskontinuierlichen Muster über die Oberfläche des Verpackungsmaterials gedruckt wird.

15. Verfahren nach Anspruch 1, bei dem das Drucken Rotationstiefdruck, Flexodruck oder lithographischer Druck ist.

16. Verfahren nach Anspruch 1, bei dem die Dispersion auf die besagte Oberfläche in einem Verhältnis von 0,163 $g/m^2$ bis 3,25 $g/m^2$ (0,1 lbs./3.000 ft. square bis 2 lbs./3.000 ft. square) gedruckt wird.

17. Verfahren nach Anspruch 1, bei dem die Beschichtungsschicht eine Stärke von 2 Mikrometer bis 20 Mikrometer hat.

18. Verfahren nach Anspruch 1, bei dem die Beschichtungsschicht eine Stärke von 2 Mikrometer bis 8 Mikrometer hat.

19. Verfahren nach Anspruch 1, bei dem das Verpackungsmaterial ein Polymerfilm ist.

20. Verfahren nach Anspruch 1, bei dem das Verpackungsmaterial ausgewählt ist aus der Gruppe bestehend aus Cellophanen, Vinylchloriden, Vinylchlorid-Copolymeren, Celluloseacetat-Filmen, Vinylidenchloriden, Vinylidenchlorid-Copolymeren, Ethylzellulose, Aluminiumfolien, Methylzellulose, Laminaten, Polyestern, Papieren, Polyethylenen, Pappe/Karton, Polypropylenen, Pergaminen, Polystyrolen, Nylons und Kombinationen davon.

**EP 1 458 495 B1**

**21.** Verfahren nach Anspruch 1, bei dem die Dispersion wässrig ist, das Polymer Lucidene enthält, und die Zeolithe eine Teilchengröße von 5 Mikrometer und eine Porengröße von 4 Angström haben.

**22.** Verfahren nach Anspruch 1, bei dem die Dispersion eine wasserbasierte Dispersion ist, das Polymer Polyamide und Nitrocellulose enthält, und die Zeolithe eine Teilchengröße von 5 Mikrometer und eine Porengröße von 4 Angström haben.

**23.** Verpackungsmaterial mit antimikrobiellen Eigenschaften, erhältlich durch das Verfahren nach Anspruch 1.

**24.** Im wesentlichen inerte wasserbasierte oder lösungsmittelbasierte Dispersion antimikrobieller Zeolithe, welche Dispersion ein Acrylpolymer und Zeolithe enthält, die antimikrobielle Metallionen enthalten, wobei das Acrylpolymer eine Säurezahl kleiner als 200 hat, wenn die Dispersion wasserbasiert ist, und wobei die Zeolithe eine Teilchengröße zwischen 2 und 5 Mikrometer, eine Porengröße zwischen 3 und 5 Angström haben und in einem Gewichtsanteil von 5 % bis 10 % in der Dispersion enthalten sind.

**25.** Dispersion nach Anspruch 24, bei der das antimikrobielle Metallion ein Silberion ist.

**26.** Dispersion nach Anspruch 24, bei der die Dispersion eine lösungsmittelbasierte Dispersion ist und das Polymer weiter ein aus der Gruppe bestehend aus Polyamiden, Polyvinylchlorid, Methyl-Methacrylaten, Ethylzellulose, Polyvinylbutyral, Polyketonen und Nitrozellulosen ausgewähltes Element enthält.

**27.** Dispersion nach Anspruch 24, bei der die Dispersion eine wasserbasierte Dispersion ist und das Polymer weiter ein aus der Gruppe bestehend aus sulfonierten Polyestern, Polyamiden, Schellacken und Maleinen ausgewähltes Element enthält.

**Revendications**

**1.** Procédé d'application d'un traitement antimicrobien sur un matériau d'emballage ayant au moins une surface, ledit procédé comprenant les étapes consistant à :

a) fournir une dispersion à base d'eau ou à base de solvant sensiblement inerte comprenant un polymère acrylique et des zéolites contenant des ions métalliques antimicrobiens, ledit polymère acrylique ayant un indice d'acidité inférieur à 200 lorsqu'une dispersion à base d'eau est utilisée, lesdites zéolites ayant une taille de particule comprise entre 2 et 5 microns, une taille de pore comprise entre 3 et 5 Angströms, et comprenant de 0,5 % à 10 % en poids de la dispersion,
b) imprimer ladite dispersion sur ladite surface dudit matériau d'emballage, et
c) sécher ladite dispersion pour former une couche de revêtement antimicrobien ayant une surface exposée contenant ledit polymère et lesdites zéolites présents sur au moins une partie de celle-ci.

**2.** Procédé selon la revendication 1, dans lequel l'ion métallique antimicrobien est un ion argent.

**3.** Procédé selon la revendication 1, dans lequel la dispersion a une viscosité comprise entre 10 et 400 centipoises à une température de 10 à 25 °C.

**4.** Procédé selon la revendication 1, dans lequel la dispersion a une viscosité comprise entre 400 et 50 000 centipoises.

**5.** Procédé selon la revendication 1, dans lequel les zéolites constituent de 1 % à 5 % en poids de la dispersion.

**6.** Procédé selon la revendication 5, dans lequel les zéolites comprennent de préférence de 2 % à 5 % en poids de la dispersion.

**7.** Procédé selon la revendication 1, dans lequel la couche de revêtement séchée est hydrophobe.

**8.** Procédé selon la revendication 1, dans lequel la dispersion est une dispersion à base de solvant et le polymère comprend en outre un élément sélectionné dans le groupe constitué de poly(amides), de poly(chlorure de vinyle), de méthacrylates de méthyle, d'éthyl cellulose, de poly(vinylbutyral), de poly(cétones) et de nitrocelluloses.

**9.** Procédé selon la revendication 1, dans lequel la dispersion est une dispersion à base d'eau, et le polymère comprend en outre un élément choisi dans le groupe constitué de poly(esters) sulfonés, de poly(amides), de gommes laques et de maléiques.

**10.** Procédé selon la revendication 9, dans lequel le polymère comprend en outre un poly(ester).

**11.** Procédé selon la revendication 10, dans lequel le polymère comprend en outre un poly(ester) sulfoné.

**12.** Procédé selon la revendication 8, dans lequel le polymère comprend en outre un poly(amide).

**13.** Procédé selon la revendication 12, dans lequel les zéolites ont une taille de particule d'au moins 5 microns, et une taille de pore d'au moins 4 Angströms.

**14.** Procédé selon la revendication 1, dans lequel la dispersion est imprimée en un motif discontinu sur la surface du matériau d'emballage.

**15.** Procédé selon la revendication 1, dans lequel l'impression est une impression en héliogravure, une impression flexographique ou une impression lithographique.

**16.** Procédé selon la revendication 1, dans lequel la dispersion est imprimée sur ladite surface à une vitesse de 0,163 g/m$^2$ à 3,25 g/m$^2$ (0,1 livre/3 000 pieds carrés à 2 livres/3 000 pieds carrés).

**17.** Procédé selon la revendication 1, dans lequel la couche de revêtement a une épaisseur de 2 microns à 20 microns.

**18.** Procédé selon la revendication 1, dans lequel la couche de revêtement a une épaisseur de 2 microns à 8 microns.

**19.** Procédé selon la revendication 1, dans lequel ledit matériau d'emballage est un film polymère.

**20.** Procédé selon la revendication 1, dans lequel ledit matériau d'emballage est choisi dans le groupe constitué de cellophanes, de chlorures de vinyle, de copolymères de chlorure de vinyle, de films d'acétate de cellulose, de chlorures de vinylidène, de copolymères de chlorure de vinylidène, d'éthyl cellulose, de feuilles d'aluminium, de méthyl cellulose, de stratifiés, de poly(esters), de papiers, de poly(éthylènes), de cartons, de poly(propylènes), de papiers cristal, de poly(styrènes), de nylons et de combinaisons de ceux-ci.

**21.** Procédé selon la revendication 1, dans lequel la dispersion est aqueuse, le polymère comprend du lucidène, les zéolites ont une taille de particule de 5 microns et une taille de pore de 4 Angströms.

**22.** Procédé selon la revendication 1, dans lequel la dispersion est une dispersion à base de solvant, le polymère comprend des poly(amides) et de la nitrocellulose, et les zéolites ont une taille de particule de 5 microns et une taille de pore de 4 Angströms.

**23.** Matériau d'emballage ayant des propriétés antimicrobiennes, pouvant être obtenu par le procédé selon la revendication 1.

**24.** Dispersion à base d'eau ou à base de solvant sensiblement inerte de zéolites antimicrobiennes, ladite dispersion comprenant un polymère acrylique et des zéolites contenant des ions métalliques antimicrobiens, ledit polymère acrylique ayant un indice d'acidité inférieur à 200 lorsque la dispersion est à base d'eau, lesdites zéolites ayant une taille de particule comprise entre 2 et 5 microns, une taille de pore comprise entre 3 et 5 Angströms et constituant de 5 % à 10 % en poids de la dispersion.

**25.** Dispersion selon la revendication 24, dans laquelle l'ion métallique antimicrobien est un ion argent.

**26.** Dispersion selon la revendication 24, dans laquelle la dispersion est une dispersion à base de solvant et le polymère comprend en outre un élément choisi dans le groupe constitué de poly(amides), de poly(chlorure de vinyle), de méthacrylates de méthyle, d'éthyl cellulose, de poly(vinylbutyral), de poly(cétones) et de nitrocelluloses.

**27.** Dispersion selon la revendication 24, dans laquelle la dispersion est une dispersion à base d'eau, et le polymère comprend en outre un élément choisi dans le groupe constitué de poly(esters) sulfonés, de poly(amides), de gommes laques et de maléiques.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4938958 A
- US 5556699 A
- US 6264936 B1